# EUROPÄISCHE PATENTSCHRIFT

(11) **EP 3 795 081 B1**
(45) Veröffentlichungstag und Bekanntmachung des Hinweises auf die Patenterteilung: **06.11.2024**
(21) Anmeldenummer: 19198590.2
(22) Anmeldetag: 20.09.2019
(51) Int. Cl.: A61B 6/00, G01T 1/24, A61B 6/03

(54) **COMPUTERTOMOGRAPH UND VERFAHREN ZUM BETRIEB EINES COMPUTERTOMOGRAPHEN**
COMPUTER TOMOGRAPH AND METHOD FOR OPERATING A COMPUTER TOMOGRAPH
TOMODENSITOMÈTRE ET PROCÉDÉ DE FONCTIONNEMENT D'UN TOMODENSITOMÈTRE

(43) Veröffentlichungstag der Anmeldung: 24.03.2021
(73) Patentinhaber: Siemens Healthineers AG, 91301 Forchheim (DE)
(72) Erfinder: CHAUDHURY, Shameem Kabir, 91058 Erlangen (DE); HILDERSCHEID, Thomas, 90518 Altdorf (DE); HOFMANN, Thomas, 91091 Großenseebach (DE); KREISLER, Björn, 91353 Hausen (DE); SCHRÖTER, Christian, 96050 Bamberg (DE); WÖLFEL, Stefan, 91077 Dormitz (DE)
(74) Vertreter: Siemens Healthineers Patent Attorneys

(56) Entgegenhaltungen:
- DE-A1- 102013 227 214
- US-A1- 2012 033 783
- US-A1- 2013 048 869

## Beschreibung

Die Erfindung betrifft einen Computertomographen (CT) mit einen Halterahmen und einem Drehkranz, welcher bezüglich des Halterahmens beweglich gelagert ist, wobei der Drehkranz einen Röntgendetektor mit einem Halbleitermaterial aufweist, welches in einem Gleichgewicht statistischer Besetzungszustände betriebsfähig ist, und wobei der CT eine Stromversorgung aufweist, welche dazu eingerichtet ist, in einem Betriebszustand des CT eine Menge an Komponenten des CT, welche für einen Bilderzeugungsprozess auf dem Drehkranz angeordnet sind, mit Leistung zu versorgen. Die Erfindung betrifft weiter ein Verfahren zum Betrieb eines solchen CT.

In einem modernen CT wird insbesondere ein direktwandelnder Röntgendetektor auf Basis von beispielsweise Cadmiumtellurid oder Cadmiumzinktellurid (oder vergleichbarer Halbleitermaterialien mit ähnlichen relevanten Eigenschaften) für photonenzählende Anwendungen verwendet. Ein derartiger Detektor weist nach Inbetriebnahme eine Zählratendrift auf, welche bei der Bildgebung zu Artefakten führen kann. Für eine verlässliche, hochauflösende und störungsfreie Bilderzeugung ist es notwendig, dass das Cadmiumtellurid bzw. das Cadmiumzinktellurid nach dem Einschaltvorgang des CT einen stabilen Gleichgewichtszustand hinsichtlich einer Besetzung von Störstellen erreicht hat.

Ein solcher Gleichgewichtszustand der Störstellenbesetzung ist dabei von der am Röntgendetektor anliegenden Spannung abhängig. Das Erreichen eines für eine artefaktfreie Bilderzeugung adäquaten Gleichgewichtszustandes nach Einschalten einer Hochspannung am Röntgendetektor ist ein Prozess, der sich über einen Zeitraum von mehreren Stunden hinziehen kann. Für einen Betrieb im medizinischen Alltag ist es aus Gründen der Effizienz wünschenswert, den CT möglichst dauerhaft betriebsbereit zur Verfügung zu haben. Dem steht die lange Dauer zum Erreichen des Gleichgewichtszustandes gegenüber, welche bei einem täglichen Einschaltvorgang im Praxisbetrieb zu erheblichen Zeitverlusten für Untersuchungen führen würde.

In DE 10 2013 227214 A1 wird ein Computertomograph mit einer Hauptstromversorgung und einer Nebenstromversorgung beschrieben. Die Hauptstromversorgung dient der Versorgung des Gesamtsystems mit Leistung während eines Betriebszustands. Die Nebenstromversorgung dient der Versorgung eines Detektors des Computertomographen in einem Ruhezustand, um den Detektor in einem Bereitschaftszustand zu halten.

Es ist Aufgabe der Erfindung, einen CT anzugeben, welcher in der Lage ist, einen Röntgendetektor mit einem in einem Gleichgewicht statistischer Besetzungszustände betriebsfähigen Halbleitermaterial möglichst schnell und einfach in einen betriebsbereiten Zustand zu versetzen. Es ist weiter Aufgabe der Erfindung, ein Verfahren zum Betrieb eines derartigen CT anzugeben.

Die erstgenannte Aufgabe wird erfindungsgemäß gelöst durch einen CT mit einen Halterahmen und einem Drehkranz, welcher bezüglich des Halterahmens beweglich gelagert ist, wobei der Drehkranz einen Röntgendetektor mit einem Halbleitermaterial aufweist, welches in einem Gleichgewicht statistischer Besetzungszustände betriebsfähig ist, und wobei der CT eine erste Stromversorgung aufweist, welche dazu vorgesehen und eingerichtet ist, in einem Betriebszustand des CT eine erste Menge an Komponenten des CT, welche für einen Bilderzeugungsprozess auf dem Drehkranz angeordnet sind, mit Leistung zu versorgen. Hierbei ist vorgesehen, dass der CT eine von der ersten Stromversorgung schaltungstechnisch trennbare zweite Stromversorgung aufweist, welche dazu vorgesehen und eingerichtet ist, in einem Ruhezustand des CT eine zweite Menge an Komponenten des CT mit Leistung zu versorgen, wobei die Komponenten der zweiten Menge dazu vorgesehen und eingerichtet sind, das Halbleitermaterial im besagten Gleichgewicht zu halten.

Weiterhin umfasst der CT eine von einer ersten Übertragungsstrecke der ersten Stromversorgung schaltungstechnisch trennbare, wenigstens teilweise am Drehkranz angeordnete zweite Übertragungsstrecke, welche mit der zweiten Stromversorgung verschaltet ist. Hierdurch sind die Übertragungsstrecken für die erste und die zweite Stromversorgung in einzelnen, voneinander trennbaren Stromkreisen realisiert, sodass die Übertragungsstrecken nicht auf verschiedene Betriebsmodi ausgelegt zu werden brauchen, sondern jede der beiden Übertragungsstrecken für die entsprechende Stromversorgung ausgelegt und entsprechend eingesetzt wird. Somit kann auf intelligente Steuerelemente zum Ab- und Zuschalten einzelner Komponenten der ersten bzw. der zweiten Menge verzichtet werden.

Hierbei weist die zweite Übertragungsstrecke einen am Drehkranz angeordneten Anschluss für ein Netzkabel auf. Hierdurch lassen sich sowohl die zweite Übertragungsstrecke als auch dessen Trennung von der ersten Übertragungsstrecke besonders einfach realisieren; für die zweite Stromversorgung wird einfach das betreffende Netzkabel an den Anschluss angeschlossen.

Vorteilhafte und teils für sich gesehen erfinderische Weiterbildungen sind Gegenstand der Unteransprüche sowie der anschließenden Beschreibung.

Die zweite Aufgabe wird erfindungsgemäß gelöst durch ein Verfahren zum Betrieb eines CT, welcher einen Halterahmen und einen bezüglich des Halterahmens beweglich gelagerten Drehkranz umfasst, wobei der Drehkranz einen Röntgendetektor mit einem Halbleitermaterial aufweist, welches in einem Gleichgewicht statistischer Besetzungszustände betriebsfähig ist. Verfahrensgemäß ist vorgesehen, dass in einem Betriebszustand des CT eine auf dem Drehkranz angeordnete erste Menge an Komponenten durch eine erste Stromversorgung mit Leistung versorgt wird, und mittels Komponenten der ersten Menge ein medizinischer Bildgebungsprozess durchgeführt wird, und dass in einem Ruhezustand des CT eine auf dem Drehkranz angeordnete zweite Menge an Komponenten durch eine von der ersten Stromversorgung schaltungstechnisch getrennte zweite Stromversorgung mit Leistung versorgt wird, und mittels der Komponenten der zweiten Menge das Halbleitermaterial im besagten Gleichgewicht gehalten wird.

Die Stromversorgung der zweiten Menge an Komponenten erfolgt im Ruhezustand über eine von einer ersten Übertragungsstrecke der ersten Stromversorgung schaltungstechnisch trennbare, wenigstens teilweise am Drehkranz angeordnete zweite Übertragungsstrecke, welche mit der zweiten Stromversorgung verschaltet ist und einen am Drehkranz angeordneten Anschluss für ein Netzkabel aufweist.

Das erfindungsgemäße Verfahren teilt die Vorzüge des erfindungsgemäßen CT. Die für das CT und für seine Weiterbildungen angegebenen Vorteile können sinngemäß auf das Verfahren übertragen werden.

Das Halbleitermaterial des Röntgendetektors ist hierbei das Material, auf welches eine Röntgenstrahlung auftrifft, die von einer Röntgenquelle des CT für eine Untersuchung eines menschlichen Körperteils emittiert wird und dabei teilweise von diesem absorbiert und/oder gestreut wird. Diese auf dem Halbleitermaterial auftreffende Röntgenstrahlung wird durch selbiges, u.a. durch Streuprozesse wie dem Compton-Effekt sowie durch den photoelektrischen Effekt, insbesondere in ein Strom- bzw. Spannungssignal umgewandelt. Unter der Betriebsfähigkeit des Halbleitermaterials ist hierbei ein Zustand zu verstehen, in welchem mit dem CT ein Aufnahmevorgang von zumindest ausreichender Bildqualität und Auflösung für einen medizinischen Bildgebungsprozess durchgeführt werden kann.

Als Gleichgewicht statistischer Besetzungszustände ist hierbei unter anderem eine definierte Verteilung von Störstellen und/oder Ladungsträgern im Halbleitermaterial umfasst. Insbesondere ist hierbei auch ein solcher Gleichgewichtszustand umfasst, welcher ein elektrodynamisches Gleichgewicht aufweist. Das Gleichgewicht statistischer Besetzungszustände umfasst dabei insbesondere ein Gleichgewicht Halbleitermaterial derart, dass beim Auftreffen von Röntgenstrahlung auf das Halbleitermaterial und einer daraus resultierenden, dortigen Erzeugung freier Ladungsträger diese im Wesentlichen ungehindert im Halbleitermaterial beweglich sind, wenn eine Spannung an das Halbleitermaterial angelegt wird. Insbesondere bringt im besagten Gleichgewicht die Röntgenstrahlung keine Änderung für einen internen Widerstand im Halbleiter für den durch die Bias-Spannung bedingten Fluss der erzeugten Ladungsträger mit sich.

Als Ruhezustand des CT ist insbesondere derjenige Zustand zu verstehen, in welchem die erste Stromversorgung ausgeschaltet ist. Die erste Stromversorgung versorgt im Betriebszustand insbesondere den Röntgendetektor und eine wesentliche Anzahl von weiteren Verbrauchern auf dem CT mit Leistung. Insbesondere ist unter der ersten Stromversorgung diejenige Stromversorgung zu verstehen, welche während eines Aufnahmevorganges und in zeitlicher Umgebung eines solchen die Hauptlast einer Leistungsversorgung der Verbraucher trägt.

Die erste Menge an Komponenten umfasst insbesondere wenigstens eine Röntgenquelle, z.B. eine Röntgenröhre, welche ggf. für eine Drehanode einen Anodenmotor aufweisen kann, Kühler und/oder Lüfter vorzugsweise zum Abführen einer in der Röntgenquelle erzeugten Wärmeleistung, sowie des Weiteren den Röntgendetektor, zudem mit diesem verbundene Elektronikkomponenten zur Signalauflösung und Bildverarbeitung, beispielsweise in Form von ASICs.

Die zweite Menge an Komponenten umfasst insbesondere für den Betrieb des Röntgendetektors angeordnete Komponenten wie z.B. eine Spannungsversorgung zum Erzeugen einer Bias-Spannung im Halbleitermaterial für einen Trennung von durch eine einfallende Röntgenstrahlung erzeugte Ladungsträger, ein Heizelement zum Beheizen des Halbleitermaterials und/oder ein Leuchtelement zum Bestrahlen des Halbleitermaterials, sowie ggf. eine Regeleinrichtung zum Regeln einer Temperatur oder einer Bestrahlung des Halbleitermaterials.

Insbesondere können einzelne Komponenten sowohl in der ersten Menge als auch in der zweiten Menge enthalten sein, wenn sie sowohl für eine Bilderzeugung als auch für das Aufrechterhalten des Gleichgewichts vorgesehen und eingerichtet sind. Bevorzugt wird die zweite Menge an Komponenten im Betriebszustand durch die erste Stromversorgung mit Leistung versorgt, und im Ruhezustand durch die zweite Stromversorgung mit Leistung versorgt, wobei die anderen Komponenten der ersten Menge, welche nicht unmittelbar für die Betriebsfähigkeit des Halbleitermaterials erforderlich sind (und somit insbesondere nicht in der zweiten Menge umfasst sind), lediglich von der ersten Stromversorgung im Betriebszustand mit Leistung versorgt werden, jedoch im Ruhezustand keine Leistung erhalten.

Die Betriebsfähigkeit des Halbleitermaterials, und somit des Röntgendetektors, setzt ein Gleichgewicht der statistischen Besetzungszustände, konkret der Störstellenbesetzung durch freie Ladungsträger voraus, um zu verhindern, dass neu erzeugte freie Ladungsträger, welche beim Auftreffen von Röntgenstrahlung auf das Halbleitermaterial entstehen, noch freie Störstellen besetzen und somit nicht für Zählereignisse zur Verfügung stehen, was sich negativ auf die Linearität auswirkt.

Um dies zu unterbinden, wird zum einen am Halbleitermaterial insbesondere eine Bias-Spannung angelegt, welche eine Drift der vorhandenen freien Ladungsträger im Halbleitermaterial bewirkt. Zudem wird eine "Sättigung" der Störstellen im Halbleitermaterial durch eine Erzeugung freier Ladungsträger im Material durch Bestrahlung sowie überdies ggf. mittels Heizen erreicht, wobei das Heizen die Beweglichkeit der Ladungsträger im Halbleitermaterial erhöht.

Der Gleichgewichtszustand stellt sich dabei erst nach einigen Stunden in ausreichend stabiler Art ein. Hierfür müssen die genannten Komponenten, welche zum Erreichen dieses Gleichgewichts erforderlich sind, entsprechend mit Leistung versorgt werden.

Daher könnte man geneigt sein, einen Dauerbetrieb der Stromversorgung des Röntgendetektors in Erwägung zu ziehen.

Der Röntgendetektor ist auf dem Drehkranz des CT gemeinsam mit einer Vielzahl anderer Verbraucher der ersten Menge an Komponenten angeordnet. Diese Verbraucher stellen, ausgenommen eine Anodenspannung der Röntgenröhre, eine dauerbetriebene Grundlast von mehreren kW dar. Aufgrund dieser Grundlast wäre ein ununterbrochener Betrieb des Drehkranzes, also auch nachts und an Wochenenden, zur Aufrechterhaltung des Gleichgewichtszustandes im Röntgendetektor energetisch ineffizient.

Als eine Möglichkeit, dies zu vermeiden, könnte man die Stromversorgung der Verbraucher auf dem Drehkranz derart ausgestalten, dass diese zwei Schalt- bzw. Betriebszustände aufweist, wobei im Hauptbetrieb alle Verbraucher und im Nebenbetrieb nur der Röntgendetektor mit Leistung versorgt werden. Ein derartiges Vorgehen würde jedoch erfordern, dass auf dem Drehkranz ein intelligent arbeitender Stromverteiler auf das Abschalten einzelner Verbraucher ausgelegt sein müsste, und hierbei die Unterscheidung der beiden Betriebszustände, da alle anderen Elektronik-Komponenten im Nebenbetrieb abgeschaltet wären, in einer eigens hierfür eingerichteten Steuerelektronik selbst übernehmen müsste.

Diese Steuerelektronik müsste ihrerseits in die Kommunikation der anderen Elektronik-Komponenten eingebunden werden, wobei zu berücksichtigen wäre, dass beim Übergang vom Neben- in den Hauptbetrieb die übrigen Komponenten erst ihren Systemstart durchlaufen, bevor sie voll kommunikationsfähig sind, es also zu Signalfehlern kommen könnte, während die Steuerelektronik der Stromversorgung bereits in Betrieb ist. Eine derartige Lösung über eine Aufteilung der Stromversorgung wäre somit störungsanfällig, was nicht zuletzt vor dem Hintergrund der hohen Anforderung eines besonders stabilen Betriebs im medizinischen Bereich, was sich auch in Zulassungsverfahren widerspiegeln, zum Verwerfen einer solchen Lösungsvariante führt.

Der Erfindung liegt nun die Überlegung zugrunde, dass das Halbleitermaterial des Röntgendetektors am einfachsten in einen betriebsbereiten Zustand zu versetzen ist, wenn der Gleichgewichtszustand dauerhaft aufrecht erhalten wird. Dies impliziert eine dauerhafte Leistungsversorgung zumindest des Röntgendetektors, da der Gleichgewichtszustand insbesondere durch eine Bias-Spannung, welche am Halbleitermaterial angelegt wird, und/oder durch ein Heizen und/oder Bestrahlen des Halbleitermaterials aufrecht erhalten werden kann.

Infolge des beschriebenen, komplexen Zusammenspiels unterschiedlicher elektrischer Verbraucher im einem CT, welche zudem teilweise zur Bilderzeugung und -verarbeitung miteinander kommunizieren, ist eine Aufteilung der Stromversorgung in einzeln zu- und abschaltbare Versorgerelemente nicht wünschenswert, da dies insbesondere einen negativen Einfluss auf die gemeinsame Zeitsynchronisation und somit die Kommunikation von bildverarbeitenden Elektronikkomponenten haben könnte.

Erfindungsgemäß wird demgegenüber nun eine zweite Stromversorgung vorgeschlagen, welche vorrangig dazu eingerichtet ist, das Halbleitermaterial des Röntgendetektors im Ruhezustand der ersten Stromversorgung in einem betriebsfähigen Zustand zu halten.

Die zweite Stromversorgung umfasst dabei insbesondere eine zweite Übertragungsstrecke, welche dazu eingerichtet ist, Leistung für die zweite Stromversorgung vom Halterahmen auf den Drehkranz zu übertragen. Bevorzugt ist hierbei die zweite Übertragungsstrecke schaltungstechnisch trennbar von einer ersten Übertragungsstrecke, welche dazu eingerichtet ist, Leistung für die erste Stromversorgung vom Halterahmen auf den Drehkranz zu übertragen. Die erste Übertragungsstrecke und die zweite Übertragungsstrecke sind hierbei in ihren jeweiligen Komponenten zumindest auf dem Drehkranz bevorzugt räumlich getrennt voneinander angeordnet, und schaltungstechnisch trennbar, indem zumindest ihre Stromkreise auf dem Drehkranz durch eine entsprechende Schaltelemente voneinander getrennt werden können.

Die erste Übertragungsstrecke umfasst dabei insbesondere induktive Elemente, wie z.B. Übertragungsspulen, zur induktiven und insbesondere kontaktlosen Leistungsübertragung vom Halterahmen auf den Drehkranz, und/oder einen Schleifring sowie entsprechend Schleifkontakte zur besagten Leistungsübertragung. Vorzugsweise umfasst die zweite Übertragungsstrecke ebenfalls induktive Elemente der beschriebenen Art. In jedem Fall umfasst die zweite Übertragungsstrecke einen am Drehkranz angeordneten Anschluss für ein Netzkabel. Für die Verwendung des Anschlusses wird bevorzugt der Drehkranz in eine Ruheposition bewegt, sodass der Anschluss für das zu verbindende Netzkabel zugänglich ist.

Bevorzugt umfasst der CT eine unterbrechungsfreie Stromversorgung (USV), welche mit der zweiten Menge an Komponenten und besonders bevorzugt mit der zweiten Stromversorgung verschaltbar ist. Die USV ist hierbei dazu vorgesehen und eingerichtet, bei einem Stromausfall oder bei kurzfristigen Leistungsschwankungen, wie sie im Krankenhausumfeld infolge stark schwankender Verbraucher auftreten können, die Komponenten der zweiten Menge weiter mit Leistung zu versorgen. Hierdurch kann sichergestellt werden, dass ein Stromausfall im Netz oder Leistungsschwankungen nicht zu einer Störung des Gleichgewichts führen, welches im Halbleitermaterial für dessen Betriebsfähigkeit aufrecht zu erhalten ist. Die USV kann hierbei insbesondere am Halterahmen angeordnet sein und wenigstens im Fall eines Einsatzes mit der zweiten Stromversorgung verschaltet werden, sodass die Verschaltung der USV mit den Komponenten der zweiten Menge insbesondere über die zweite Stromversorgung erfolgen kann.

Günstigerweise umfasst die zweite Menge an Komponenten eine erste Spannungsversorgung zum Anlegen einer Bias-Spannung an das Halbleitermaterial. Die erste Spannungsversorgung weist dabei insbesondere Schaltelemente zum Aufbereiten der Bias-Spannung aus der Leistung der zweiten Stromversorgung, Verbindungselemente zum Verbinden mit dem Halbleitermaterial und zum Anlegen der aufbereiteten Bias-Spannung an dieses, sowie ggf. Mess- und/oder Regelelemente zum Überprüfen bzw. zum Regeln der an das Halbleitermaterial angelegten Bias-Spannung auf. Bevorzugt ist die erste Spannungsversorgung am Drehkranz angeordnet, und ist überdies besonders bevorzugt mit der zweiten Stromversorgung verschaltet oder verschaltbar, also über ein Schaltelement mit dieser verbindbar. Die Bias-Spannung sorgt für eine entsprechende Drift der freien Leitungsträger im Halbleitermaterial, sodass diese dort die Störstellen besetzen, wodurch freie Ladungsträger, welche durch eine auf das Halbleitermaterial auftreffende Röntgenstrahlung erzeugt werden, im Halbleitermaterial eine höhere Beweglichkeit aufweisen, und insbesondere dort nicht die bereits abgesättigten Störstellen besetzen. Dies erhöht die Linearität des Detektors. Da es bis zu einigen Stunden dauern kann, mittels der Bias-Spannung das Halbleitermaterial in ein Gleichgewicht hinsichtlich der Störstellenbesetzung zu bringen, ist es somit besonders vorteilhaft, die Bias-Spannung mittels der ersten Spannungsversorgung dauerhaft an das Halbleitermaterial anzulegen.

Bevorzugt umfasst der CT eine von der ersten Stromversorgung und der zweiten Stromversorgung schaltungstechnisch trennbare dritte Stromversorgung, welche insbesondere mit der ersten Spannungsversorgung und/oder der zweiten Stromversorgung verschaltbar ist, und welche dazu vorgesehen und eingerichtet ist, bei einem Systemneustart des CT die erste Spannungsversorgung für eine Aufrechterhaltung der Bias-Spannung am Halbleitermaterial während des Systemneustarts mit Leistung zu versorgen. Die dritte Stromversorgung umfasst hierbei insbesondere einen auf dem Drehkranz angeordneten Leistungspuffer, bevorzugt in Form einer Batterie oder eines Akkumulators, welcher beispielsweise im Betriebszustand der CT durch die erste Stromversorgung gespeist und beladen werden kann. Die dritte Stromversorgung kann aber insbesondere auch durch einen eigenständig schaltbaren Stromkreis innerhalb der zweiten Stromversorgung implementiert sein, und sich somit insbesondere deren zweiter Übertragungsstrecke bedienen. Insbesondere ist die dritte Stromversorgung dazu eingerichtet, die Bias-Spannung auch während Wartungsarbeiten an sonstigen Komponenten der zweiten Gruppe aufrecht zu erhalten, wenn die zweite Stromversorgung für die Wartungsarbeiten unterbrochen wird.

Aus Gründen der besseren Synchronisierung der Kommunikation verschiedener bildgebender und insbesondere signalverarbeitender Komponenten ist es vorteilhaft, in regelmäßigen Abständen, z.B. ein Mal am Tag, einen Systemneustart des CT durchzuführen. Hierbei wird insbesondere die Zeitsynchronisierung der einzelnen Komponenten neu durchgeführt, was sich positiv auf die Stabilität der Bilderzeugung und - verarbeitung auswirkt. Bei einem derartigen Systemneustart wird üblicherweise die komplette Stromversorgung aller Komponenten des CT unterbrochen. Durch die wie beschrieben ausgestaltete dritte Stromversorgung kann insbesondere die erste Spannungsversorgung während des Systemneustarts weiterhin mit Leistung versorgt werden, sodass der Systemneustart nicht zu einem Spannungsverlust im Halbleitermaterial führt.

In einer vorteilhaften Ausgestaltung umfasst das CT einen zwischen die USV und die erste Spannungsversorgung geschalteten Spannungspuffer, welcher zum Ausgleich einer Spannungsschwankung bei einem Einsatz der USV vorgesehen und eingerichtet ist. Wird der Einsatz der USV erforderlich, beispielsweise infolge eines Stromausfalls oder infolge von erheblichen Spannungsschwankungen im Netz, so kann es im CT durch das Umschalten auf die Leistungsversorgung der USV zu Spannungsschwankungen kommen. Um diese auszugleichen, wird der ersten Spannungsversorgung ein Spannungspuffer vorgeschaltet, welcher einerseits durch den Umschaltvorgang entstehende Spannungs- und/oder Leistungsschwankungen an einem Eingang der ersten Spannungsversorgung ausgleicht, und andererseits eine eventuelle Anlaufzeit der USV überbrückt, bis diese voll einsatzfähig ist. Insbesondere kann der Spannungspuffer auch durch den Leistungspuffer der dritten Stromversorgung gegeben sein.

Günstigerweise umfasst die zweite Menge an Komponenten ein Heizelement zum Beheizen des Halbleitermaterials und/oder ein Leuchtelement zum Bestrahlen des Halbleitermaterials. Unter einem Bestrahlen des Halbleiermaterials mittels des Leuchtelements ist hierbei insbesondere zu verstehen, dass der CT über die Röntgenquelle hinaus ein Leuchtelement verfügt, welches dazu eingerichtet ist, das Halbleitermaterial zu bestrahlen, während die Röntgenquelle insbesondere keine Strahlung emittiert.

Durch das zusätzliche Bestrahlen des Halbleitermaterials können in diesem freie Ladungsträger erzeugt werden, welche die Störstellen des Halbleitermaterials besetzen können, und somit zum Einstellen des für die Betriebsfähigkeit erforderlichen Gleichgewichtes beitragen Ein Heizen erhöht die Beweglichkeit freier Ladungsträger im Halbleitermaterial. Hierdurch stellt sich das angestrebte Gleichgewicht leichter und insbesondere schneller ein, und bleibt insbesondere auch aufrecht erhalten, wenn die Röntgenquelle über einen längeren Zeitraum hinweg keinerlei Strahlung auf das Halbleitermaterial emittiert und infolgedessen keine freien Ladungsträger durch Röntgenstrahlung erzeugt werden.

Das Heizelement umfasst dabei insbesondere einen ohmschen Widerstand, welcher zum kontrollierten Umwandeln eines elektrischen Stroms in Wärme vorgesehen und eingerichtet ist. Das Leuchtelement umfasst hierbei bevorzugt eine Lichtquelle, welche dazu eingerichtet ist, Licht im sichtbaren Bereich und/oder im UV-Bereich und/oder im IR-Bereich zu erzeugen. Das Leuchtelement kann dabei eine Anzahl an LEDs aufweisen.

Günstigerweise ist die zweite Stromversorgung derart ausgestaltet, dass bei einem Systemneustart des Computertomographen die Leistungsversorgung der zweiten Menge an Komponenten unterbrochen wird. Insbesondere bedeutet dies, dass die erste Spannungsversorgung zum Bereitstellen der Bias-Spannung für das Halbleitermaterial während des Systemneustarts von der dritten Stromversorgung gespeist wird. Hierdurch können auch das Heizelement bzw. das Leuchtelement und mit ihnen ggf. vorhandene Regeleinrichtungen zum Regeln einer Temperatur bzw. einer Bestrahlung des Halbleitermaterials neu gestartet werden.

Bevorzugt umfasst der CT eine erste Regeleinrichtung zum Regeln einer Temperatur des Halbleitermaterials, wobei die erste Regeleinrichtung ein erstes nicht-flüchtig speicherndes Regelelement aufweist, welches dazu vorgesehen und eingerichtet ist, bei einem Trennen der ersten Regeleinrichtung von einer Leistungsversorgung einen vorliegenden Sollwert und/oder einen vorliegenden Istwert einer Temperatur zu speichern, und bei einer erneuten Leistungsversorgung der ersten Regeleinrichtung den gespeicherten Sollwert bzw. Istwert in der ersten Regeleinrichtung zur Verfügung zu stellen. Das erste Regelelement weist hierzu bevorzugt einen entsprechenden Elektronikbaustein auf, welcher zum nicht-flüchtigen Speichern des vorliegenden Sollwertes und/oder Istwertes der Temperatur unabhängig von einer Leistungsversorgung eingerichtet ist, sodass der besagte Elektronikbaustein den jeweils gespeicherten Wert nach einem erneuten Verbinden mit der Leistungsversorgung bereitstellt. Bei einem Systemneustart muss somit die erste Regeleinrichtung die Temperatur nicht von Grund auf neu regeln, sondern kann die Regelung mit den Werten fortsetzen, welche ihr vor dem Systemneustart und der damit verbundenen Trennung der ersten Regeleinrichtung von der Leistungsversorgung vorlagen.

Als weiter vorteilhaft erweist es sich, wenn der CT eine zweite Regeleinrichtung zum Regeln einer Bestrahlung des Halbleitermaterials aufweist, wobei die zweite Regeleinrichtung ein zweites nicht-flüchtig speicherndes Regelelement aufweist, welches dazu vorgesehen und eingerichtet ist, bei einem Trennen der zweiten Regeleinrichtung von einer Leistungsversorgung einen vorliegenden Sollwert und/oder einen vorliegenden Istwert einer Bestrahlung zu speichern, und bei einer erneuten Leistungsversorgung der zweiten Regeleinrichtung den gespeicherten Sollwert bzw. Istwert in der zweiten Regeleinrichtung zur Verfügung zu stellen. Ein Sollwert bzw. Istwert einer Bestrahlung kann hierbei eine Bestrahlungsstärke oder eine hierzu vergleichbare physikalische Strahlungsgröße und/oder eine Frequenz oder eine hierzu vergleichbare spektrale Kenngröße betreffen. Eine Regelung der Bestrahlung kann insbesondere auch anhand der durch die Bestrahlung im Halbleitermaterial erzeugten und entsprechend als ein Sensorstrom ermittelten freien Ladungsträger erfolgen.

Das zweite Regelelement weist hierzu bevorzugt einen entsprechenden Elektronikbaustein auf, welcher zum nicht-flüchtigen Speichern des vorliegenden Sollwertes und/oder Istwertes der Bestrahlung unabhängig von einer Leistungsversorgung eingerichtet ist, sodass der besagte Elektronikbaustein den jeweils gespeicherten Wert nach einem erneuten Verbinden mit der Leistungsversorgung bereitstellt. Bei einem Systemneustart muss somit die zweite Regeleinrichtung die Bestrahlung nicht von Grund auf neu regeln, sondern kann die Regelung mit den Werten fortsetzen, welche ihr vor dem Systemneustart und der damit verbundenen Trennung der zweiten Regeleinrichtung von der Leistungsversorgung vorlagen.

Günstigerweise ist die zweite Menge an Komponenten im Drehkranz elektrisch isoliert angeordnet. Insbesondere sind die Komponenten der zweiten Menge derart isoliert am Drehkranz angeordnet, dass die Komponenten der ersten Menge, welche nicht Teil der zweiten Menge sind, also z.B. eine Röntgenquelle oder Kühler bzw. Lüfter, im Ruhezustand strom- und spannungsfrei bleiben, und somit im Ruhezustand gewartet oder ausgetauscht werden können. Hierbei verbleibt durch die zweite Stromversorgung die zweite Menge an Komponenten mit Leistung versorgt. Eine besagte Wartung bzw. ein Austausch der Komponenten nur der ersten Menge kann somit derart durchgeführt werden, dass das Halbleitermaterial weiter betriebsfähig bleibt, und der CT unmittelbar nach Beendigung der Wartung bzw. des Austausches, insbesondere ohne weitere Wartezeiten, wieder voll einsatzfähig ist.

Zweckmäßigerweise werden für das Verfahren im Betriebszustand eine Temperatur des Halbleitermaterials und/oder eine Bestrahlung des Halbleitermaterials zur Aufrechterhaltung des Gleichgewichts mit einer ersten Genauigkeitskonstante geregelt, wobei in einem erweiterten Ruhemodus die Temperatur bzw. die Bestrahlung des Halbleitermaterials mit einer zweiten Genauigkeitskonstante geregelt werden, welche größer ist, als die erste Genauigkeitskonstante. Die zweite Genauigkeitskonstante ist hierbei und im Folgenden dann größer als die erste Genauigkeitskonstante zu bezeichnen, wenn durch die zweite Genauigkeitskonstante eine größere Ungenauigkeit toleriert wird, und somit die Regelung im erweiterten Ruhemodus "gröber" ist und insbesondere mit mehr potentieller Abweichung von einem vorgesehenen Sollwert erfolgen kann, als im Betriebszustand. Als erste bzw. zweite Genauigkeitskonstante ist bevorzugt eine maximale Abweichung von einem Sollwert umfasst, wobei die maximale Abweichung vom Sollwert bestimmt, wann aktiv durch eine Stellgröße in den betreffenden Regelkreis eingegriffen wird.

Als weiter vorteilhaft erweist es sich für das Verfahren, wenn im Betriebszustand eine Temperatur des Halbleitermaterials und/oder eine Bestrahlung des Halbleitermaterials zur Aufrechterhaltung des Gleichgewichts mit einer ersten Genauigkeitskonstante geregelt werden, und wenn dabei vor einem Systemneustart des CT für die Regelung jeweils durch ein entsprechendes, nicht-flüchtig speicherndes Regelelement ein Sollwert und/oder ein Istwert der Temperatur bzw. der Bestrahlung gespeichert wird, und nach dem Systemneustart für die Regelung zur Verfügung gestellt wird. Hierdurch kann die Regelung der Temperatur der Bestrahlung unmittelbar nach dem Systemneustart mit hoher Stabilität fortgesetzt werden. Da die Zeitdauer für einen Systemneustart eher im Bereich weniger Minuten liegt, kann das Halbleitermaterial während des Systemneustarts unter den anzunehmenden Bedingungen nur um wenige Grad abkühlen, bis der Systemneustart vollzogen ist, und die Regelung fortgesetzt werden kann. Die Bestrahlung kann dabei insbesondere anhand einer instantan gemessenen Bestrahlungsstärke oder auch anhand eines gemessenen Stromes an freien Ladungsträgern geregelt werden. Aus diesem Grund ist eine Fortsetzung der Regelung insbesondere mit den Temperaturwerten bzw. den Bestrahlungswerten vorteilhaft, welche vor dem Systemneustart vorlagen.

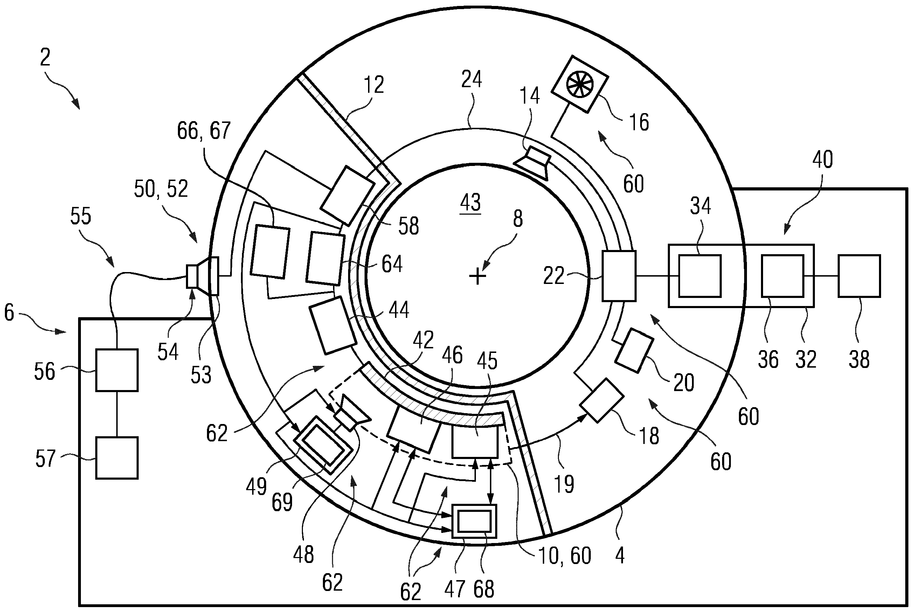

Nachfolgend wird ein Ausführungsbeispiel der Erfindung anhand einer Zeichnung näher erläutert. Hierbei zeigt schematisch: FIG 1 eine Querschnittdarstellung eines CT mit einer zweiten Stromversorgung für den auf dem Drehkranz angeordneten Röntgendetektor.

In FIG 1 ist schematisch eine Querschnittdarstellung eines CT 2 dargestellt. Der CT 2 umfasst dabei einen Drehkranz 4 und einen Halterahmen 6. Der Drehkranz 4 ist hierbei bezüglich des Halterahmens 6 um eine senkrecht zur Bildebene stehende Achse 8 im Zentrum des Drehkranzes 4 drehbar gelagert. Auf dem Drehkranz 4 ist der Röntgendetektor 10 in einer elektrischen Isolierung 12 angeordnet.

Auf dem Drehkranz 4 sind eine Röntgenquelle 14, ein Lüfter 16, eine Bildverarbeitung 18 und eine Steuerelektronik 20 mit einem zentralen Stromverteiler 22 leitend verbunden, von welchem aus auch eine Leitung 24 zur Versorgung des Röntgendetektors 10 abführt. Die Bildverarbeitung 18 ist über ein optisches Signalkabel 19 mit dem Röntgendetektor 10 verbunden. In der Röntgenquelle 14 sind weiter in nicht näher dargestellter Weise eine Drehanode mitsamt Antrieb, ein Kathodenheizer und ein Kühler angeordnet. Der zentrale Stromverteiler 22 ist verbunden mit einer ersten Übertragungsstrecke 32, welche eine Energie aufnehmende Komponente 34 aufweist, die Leistung zum zentralen Stromverteiler 22 führt.

Eine auf dem Halterahmen 6 angeordnete Energie abgebende Komponente 36 der ersten Übertragungsstrecke 32 ist mit einer ersten Leistungsquelle 38 verbunden, welche beispielsweise durch einen Netzanschluss gegeben sein kann. Die erste Leistungsquelle 38, die erste Übertragungsstrecke 32 und der zentrale Stromverteiler 22 bilden zusammen mit den entsprechenden Verbindungsleitungen die wesentlichen Komponenten einer ersten Stromversorgung 40. Die Energie aufnehmende Komponente 34 und die Energie abgebende Komponente 36 können dabei jeweils als Spulen eines Spulenpaars für eine induktive Energieübertragung ausgebildet sein, oder als ein Schleifring mit einem Schleifringkontakt.

Der Röntgendetektor 10 weist Schicht eines Halbleitermaterials 42 auf, welche derart angebracht ist, dass eine von der Röntgenquelle 14 erzeugte (und ggf. durch ein im Innenraum 43 des Drehkranzes 4 positioniertes Objekt teilweise gestreute und/oder absorbierte) Röntgenstrahlung (nicht näher dargestellt) auf dem Halbleitermaterial 42 auftrifft. Das Halbleitermaterial kann hierbei insbesondere durch Cadmiumtellurid oder Cadmiumzinktellurid, oder auch durch einen vergleichbaren Halbleiter mit ähnlichen relevanten Eigenschaften gegeben sein.

Das Halbleitermaterial 42 ist mit einer ersten Spannungsversorgung 44 derart verschaltet, dass durch die erste Spannungsversorgung 44 eine Bias-Spannung an das Halbleitermaterial 42 angelegt werden kann. Weiter ist das Halbleitermaterial thermisch mit einem Heizelement 45 und einem Kühlelement 46 gekoppelt. Das Heizelement 45 ist dazu eingerichtet, das Halbleitermaterial 42 zu beheizen, um dort die Beweglichkeit freier Ladungsträger zu erhöhen. Hierdurch wird einerseits eine Sättigung der Störstellen im Halbleitermaterial 42 erleichtert, andererseits die Linearität für freie Ladungsträger erhöht, welche durch von der Röntgenquelle 14 emittiert Röntgenstrahlung im Halbleitermaterial 42 erzeugt werden. Das Kühlelement 46 ist dazu eingerichtet, die Temperatur des Halbleitermaterials 42 zu verringern, wenn diese infolge einer einfallenden Röntgenstrahlung einen kritischen Wert einnimmt.

Das Heizelement 45 und das Kühlelement 46 sind mit einer ersten Regeleinrichtung 47 verbunden, welche dazu eingerichtet ist, die Temperatur des Halbleitermaterials 42 zu regeln, und hierbei insbesondere durch ein Heizen die Beweglichkeit freier Ladungsträger weiter ermöglicht, wenn die erste Stromversorgung 40 abgeschaltet ist. Hierfür ist die erste Regeleinrichtung 47 mit einem nicht näher dargestellten Temperatursensor verbunden, welcher eine Temperatur des Halbleitermaterials 42 misst.

In unmittelbarer Nähe des Halbleitermaterials 42 ist auf dem Drehkranz ein Leuchtelement 48 angeordnet, welches z.B. durch eine Anzahl an LEDs implementiert sein kann, und dazu eingerichtet ist, durch eine Bestrahlung des Halbleitermaterials 42 in diesem freie Ladungsträger zu erzeugen, welche insbesondere durch das Anlegen der von der ersten Spannungsversorgung 44 bereitgestellten Bias-Spannung an das Halbleitermaterial 42 in diesem dessen Störstellen besetzen. Dies hat zur Folge, dass weitere freie Ladungsträger, welche durch eine von der Röntgenquelle 14 emittierte und auf dem Halbleitermaterial 42 auftreffende Röntgenstrahlung in diesem erzeugt werden, ein besonders lineares Verhalten hinsichtlich der Detektion der Röntgenstrahlung ermöglichen, da die Störstellen in einem sich infolge der Bias-Spannung einstellenden Gleichgewicht abgesättigt sind, und das Halbleitermaterial 42 dadurch voll betriebsfähig ist.

Die Bestrahlung des Halbleitermaterials 42 wird dabei über eine zweite Regeleinrichtung 49 geregelt, welche hierfür mit einem nicht näher dargestellten, in unmittelbarer Nähe des Halbleitermaterials 42 angeordneten Lichtsensor verbunden ist, der eine Bestrahlungsstärke misst und so Rückschlüsse auf eine Bestrahlungsstärke am Halbleitermaterial 42 zulässt.

Die erste Stromversorgung 40 ist über den zentralen Stromverteiler 22 mit der Röntgenquelle 14, dem Lüfter 16, der Steuerelektronik 20 und der Bildverarbeitung 18 verbunden, sowie über die Leitung 24 und einen an diese angeschlossenen Schutzschalter 58 mit der ersten Spannungsversorgung 44, dem Heizelement 45, dem Kühlelement 46, der ersten Regeleinrichtung 47, dem Leuchtelement 48 und der zweiten Regeleinrichtung 49 verschaltbar. Im Betriebszustand des CT 2 beziehen die besagten, am Schutzschalter 58 verschalteten Komponenten ihre Leistungsversorgung über die Leitung 24 durch die erste Stromversorgung 40; der Schutzschalter 58 ist dabei in einem entsprechenden Schaltzustand, sodass ein Leistungsfluss zu den genannten Komponenten hin ermöglicht wird.

Die unmittelbar am zentralen Stromverteiler 22 (also nicht über den Schutzschalter 58) an die erste Stromversorgung 40 angeschlossenen Komponenten beziehen nur im Betriebszustand eine Leistung. Wird die erste Stromversorgung 40 unterbrochen, weil etwa der CT über Nacht in einen Ruhemodus versetzt werden soll, oder ein Systemneustart erfolgen soll, erfolgt für keine der unmittelbar am zentralen Stromverteiler 22 angeschlossenen Komponenten eine Leistungsversorgung.

Für den Ruhemodus, in welchem die erste Stromversorgung 40 abgeschaltet ist, weist der CT 2 eine zweite Stromversorgung 50 auf, welche mit dem Schutzschalter 58 verbunden ist. Der Schutzschalter 58 stellt dabei sicher, dass für den Fall, dass die erste Stromversorgung 40 abgeschaltet ist, und eine Leistungsversorgung über die zweite Stromversorgung 50 erfolgt, kein Leistungsrückfluss zum zentralen Stromverteiler 22 erfolgt, und somit insbesondere die Röntgenquelle 14, der Lüfter 16, die Steuerelektronik 20 und die Bildverarbeitung 18 strom- und spannungsfrei bleiben, und somit im Ruhezustand gewartet oder ausgetauscht werden können. Hierfür ist zusätzlich die Isolierung 12 für die mit dem Schutzschalter 58 verschalteten Komponenten vorgesehen.

Die zweite Stromversorgung 50 weist eine zweite Übertragungsstrecke 52 mit einer am Drehkranz 4 angeordneten Energie aufnehmenden Komponente 53 und einer wenigstens teilweise am Halterahmen 6 angeordneten Energie abgebenden Komponente 54 auf. Die Energie aufnehmende Komponente 53 ist vorliegend durch einen Anschluss für einen Netzstecker gebildet. Die Energie abgebende Komponente 54 umfasst den Netzstecker, sowie ein Netzkabel 55, welches am Halterahmen 6 mit einer USV 56 verschaltet ist, die ihrerseits mit einem Netzanschluss 57 zur Verbindung mit einem nicht näher dargestellten Stromnetz verbunden ist. Die USV 56 ist dazu eingerichtet, im Falle eines Stromausfalls die zweite Stromversorgung 50 aufrecht zu erhalten. Zwar weisen Krankenhäuser oftmals eine eigene USV auf, jedoch werden CT zunehmend auch in Arztpraxen oder kleineren Behandlungszentren ohne eigene USV eingesetzt, sodass hier die USV 56 den Betrieb der zweiten Stromversorgung 50 garantiert.

Die Energie aufnehmende Komponente 53 und die Energie abgebende Komponente 54 der zweiten Übertragungsstrecke 52 können aber, alternativ zur dargestellten Ausführungsform, oder auch zusätzlich hierzu, ein Paar von Induktionsspulen für eine induktive Energieübertragung umfassen.

Im Ruhezustand wird somit im Halbleitermaterial 42 des Röntgendetektors 10 weiterhin durch die Bias-Spannung der ersten Spannungsversorgung 44, eine durch das Heizelement 45 bzw. das Kühlelement 46 eingestellte Temperatur sowie durch eine vom Leuchtelement 48 bewirkte Bestrahlung das Gleichgewicht der besetzungszustände der Störstellen aufrecht erhalten, wobei nun die Leistung über die zweite Stromversorgung 50 zugeführt wird.

Aufgrund der elektrischen Isolierung 12 und der Schutzschaltung 58 ist der Drehkranz, bis auf den Röntgendetektor 10 selbst, im Ruhezustand spannungsfrei, so dass in dieser Zeit auch Wartungs- oder Reparaturarbeiten, welche nicht den Röntgendetektor 10 betreffen, durchgeführt werden können.

Die am Drehkranz für die Bilderzeugung angeordneten Verbraucher bilden somit eine erste Menge 60 an Komponenten, welche im Betriebszustand des CT 2 von der ersten Stromversorgung 40 mit Leistung versorgt werden. Infolge der Isolierung 12 und der schaltungstechnischen Trennung mittels des Schutzschalters 58 vom zentralen Stromverteiler 22 bilden die erste Spannungsversorgung 44, das Heizelement 45, das Kühlelement 46, die erste Regeleinrichtung 47, das Leuchtelement 48 und die zweite Regeleinrichtung 49 eine zweite Menge 62 an Komponenten, welche im Ruhezustand, also bei Abschalten der ersten Stromversorgung 40, von der zweiten Stromversorgung 50 mit Leistung versorgt werden, um das Halbleitermaterial 42 des Röntgendetektors 10 möglichst in einem betriebsfähigen Zustand zu halten.

Auch im Betriebszustand wird dabei das Halbleitermaterial 42 des Röntgendetektors 10 durch die Komponenten der zweiten Menge 62 betriebsfähig gehalten, wobei ggf. während einer einzelnen Röntgenaufnahme das Leuchtelement 48 und ggf. das Heizelement 45 abgeschaltet werden kann. Im Betriebszustand können hierbei die Komponenten der zweiten Menge 62 ihre Leistung durch die erste Stromversorgung 40 beziehen, sodass die zweite Stromversorgung 50 die Leistungsversorgung der zweiten Menge 62 an Komponenten lediglich im Ruhezustand übernimmt.

Zwischen die erste Spannungsversorgung 44 und den Schutzschalter 58 (und somit auch die zweite Stromversorgung 50) ist ein Spannungspuffer 64 geschaltet, welcher bei einem Einsatz der USV 56 auftretenden Spannungsschwankungen ausgleicht, um die Bias-Spannung auch bei einem Stromausfall und dem daraus resultierenden Einsatz der USV 56 möglichst konstant zu halten.

Soll nun ein Systemneustart durchgeführt werden, wird auch die zweite Stromversorgung 50 kontrolliert für den Neustart unterbrochen. Um dennoch die Bias-Spannung aufrecht erhalten zu können, ist als eine dritte Stromversorgung 66 eine Batterie 67 mit der ersten Spannungsversorgung 44 in nicht näher dargestellter Weise schaltbar verbunden. Die dritte Stromversorgung 66 stellt während des Systemstarts die nötige Leistung bereit, um weiterhin die Bias-Spannung am Halbleitermaterial 42 anlegen zu können. Da der Systemneustart meist nur wenige Minuten dauert, kann dabei der Ausfall des Heizelements 45 und des Leuchtelements 48 infolge der vergleichsweise langsamen Temperaturrelaxation bzw. des instantanen Bestrahlens durch das Leuchtelement 48 nach dem Systemneustart vernachlässigt werden.

Um dabei die Temperaturregelung und die Regelung der Bestrahlung nach dem Systemneustart möglichst nahe der letzten Werte fortsetzen zu können, weist die erste Regeleinrichtung 47 ein erstes nicht-flüchtig speicherndes Regelelement 68 auf, welches den vor dem Systemstart vorliegenden Istwert und/oder Sollwert der Temperatur auch bei einem Unterbrechen der zweiten Stromversorgung 50, und damit der Leistungsversorgung der ersten Regeleinrichtung 47 speichert. Die Regelung der Temperatur kann dann beispielsweise mit dem zuletzt bekannten Istwert als Ausgangswert fortgesetzt werden.

Analog dazu weist die zweite Regeleinrichtung 49 ein zweites nicht-flüchtig speicherndes Regelelement 69 auf. welches den vor dem Systemstart vorliegenden Istwert und/oder Sollwert einer Bestrahlungsstärke und ggf. Frequenz bzw. Wellenlänge auch bei einem Unterbrechen der zweiten Stromversorgung 50, und damit der Leistungsversorgung der zweiten Regeleinrichtung 49 speichert. Die Regelung kann dann ebenso mit dem zuletzt bekannten Istwert als Ausgangswert fortgesetzt werden.

Insbesondere kann dabei die Regelung durch die erste Regeleinrichtung 47 und die zweite Regeleinrichtung 49 im Ruhezustand in geringem Maße "gröber" erfolgen (d.h., dass z.B. eine geringfügig höhere Abweichung vom jeweiligen Sollwert toleriert wird) als im Betriebszustand. Hierdurch wird eine Art erweiterter Ruhemodus definiert. Dies kann den Energieverbrauch senken, wobei dennoch das Halbleitermaterial 42 nahezu ideal betriebsfähig gehalten wird.

Obwohl die Erfindung im Detail durch das bevorzugte Ausführungsbeispiel näher illustriert und beschrieben wurde, ist die Erfindung nicht durch dieses Ausführungsbeispiel eingeschränkt. Andere Variationen können vom Fachmann hieraus abgeleitet werden, ohne den Schutzumfang der Erfindung zu verlassen.

## Patentansprüche

1. Computertomograph (2) mit einen Halterahmen (6) und einem Drehkranz (4), welcher bezüglich des Halterahmens (4) beweglich gelagert ist,
wobei der Drehkranz (4) einen Röntgendetektor (10) mit einem Halbleitermaterial (42) aufweist, welches in einem Gleichgewicht statistischer Besetzungszustände betriebsfähig ist,
wobei der Computertomograph (2) eine erste Stromversorgung (40) aufweist, welche dazu eingerichtet ist, in einem Betriebszustand des Computertomographen (2) eine erste Menge (60) an Komponenten des Computertomographen (2), welche für einen Bilderzeugungsprozess auf dem Drehkranz (4) angeordnet sind, mit Leistung zu versorgen,
wobei der Computertomograph (2) eine von der ersten Stromversorgung (40) schaltungstechnisch trennbare zweite Stromversorgung (50) aufweist, welche dazu eingerichtet ist, in einem Ruhezustand des Computertomographen (2) eine zweite Menge (62) an Komponenten des Computertomographen (2) mit Leistung zu versorgen, und
wobei die Komponenten der zweiten Menge (62) dazu eingerichtet sind, das Halbleitermaterial (42) im besagten Gleichgewicht zu halten,
**gekennzeichnet durch**
eine von einer ersten Übertragungsstrecke (32) der ersten Stromversorgung (40) schaltungstechnisch trennbare, wenigstens teilweise am Drehkranz (4) angeordnete zweite Übertragungsstrecke (52), welche mit der zweiten Stromversorgung (50) verschaltet ist und einen am Drehkranz (4) angeordneten Anschluss (53) für ein Netzkabel (55) aufweist.

2. Computertomograph (2) nach Anspruch 1,
umfassend eine unterbrechungsfreie Stromversorgung (56), welche mit der zweiten Menge (62) an Komponenten verschaltbar ist.

3. Computertomograph(2) nach Anspruch 1 oder Anspruch 2, wobei die zweite Menge (62) an Komponenten eine erste Spannungsversorgung (44) zum Anlegen einer Bias-Spannung an das Halbleitermaterial (42) umfasst.

4. Computertomograph (2) nach Anspruch 3,
umfassend eine von der ersten Stromversorgung (40) und der zweiten Stromversorgung (50) schaltungstechnisch trennbare dritte Stromversorgung (66), welche dazu eingerichtet ist, bei einem Systemneustart des Computertomographen (2) die erste Spannungsversorgung (44) für eine Aufrechterhaltung der Bias-Spannung am Halbleitermaterial (42) während des Systemneustarts mit Leistung zu versorgen.

5. Computertomograph (2) nach Anspruch 3 oder Anspruch 4 in Verbindung mit Anspruch 2,
umfassend einen zwischen die unterbrechungsfreie Stromversorgung (56) und die erste Spannungsversorgung (44) geschalteten Spannungspuffer (64), welcher zum Ausgleich einer Spannungsschwankung bei einem Einsatz der unterbrechungsfreien Stromversorgung (56) vorgesehen und eingerichtet ist.

6. Computertomograph (2) nach einem der vorhergehenden Ansprüche,
wobei die zweite Menge (62) an Komponenten ein Heizelement (45) zum Beheizen des Halbleitermaterials (42) und/oder ein Leuchtelement (48) zum Bestrahlen des Halbleitermaterials (42) umfasst.

7. Computertomograph (2) nach Anspruch 6,
wobei die zweite Stromversorgung (50) derart ausgestaltet ist, dass bei einem Systemneustart des Computertomographen (2) die Leistungsversorgung der zweiten Menge (62) an Komponenten unterbrochen wird.

8. Computertomograph nach Anspruch 6 oder Anspruch 7, umfassend eine erste Regeleinrichtung (47) zum Regeln einer Temperatur des Halbleitermaterials (42),
wobei die erste Regeleinrichtung (47) ein erstes nicht-flüchtig speicherndes Regelelement (68) aufweist, welches dazu eingerichtet ist, bei einem Trennen der ersten Regeleinrichtung (47) von einer Leistungsversorgung einen vorliegenden Sollwert und/oder einen vorliegenden Istwert einer Temperatur zu speichern, und bei einer erneuten Leistungsversorgung der ersten Regeleinrichtung (47) den gespeicherten Sollwert bzw. Istwert in der ersten Regeleinrichtung (47) zur Verfügung zu stellen.

9. Computertomograph (2) nach einem der Ansprüche 6 bis 8, umfassend eine zweite Regeleinrichtung (49) zum Regeln einer Bestrahlung des Halbleitermaterials (42),
wobei die zweite Regeleinrichtung (49) ein zweites nicht-flüchtig speicherndes Regelelement (69) aufweist, welches dazu eingerichtet ist, bei einem Trennen der zweiten Regeleinrichtung (49) von einer Leistungsversorgung einen vorliegenden Sollwert und/oder einen vorliegenden Istwert einer Bestrahlung zu speichern, und bei einer erneuten Leistungsversorgung der zweiten Regeleinrichtung (49) den gespeicherten Sollwert bzw. Istwert in der zweiten Regeleinrichtung (49) zur Verfügung zu stellen.

10. Computertomograph (2) nach einem der vorhergehenden Ansprüche,
wobei die zweite Menge (62) an Komponenten im Drehkranz (4) elektrisch isoliert angeordnet ist.

11. Verfahren zum Betrieb eines Computertomographen (2), welcher einen Halterahmen (6) und einen bezüglich des Halterahmens (6) beweglich gelagerten Drehkranz (4) umfasst, wobei der Drehkranz (4) einen Röntgendetektor (10) mit einem Halbleitermaterial (42) aufweist, welches in einem Gleichgewicht statistischer Besetzungszustände betriebsfähig ist, wobei in einem Betriebszustand des Computertomographen (2) eine auf dem Drehkranz (4) angeordnete erste Menge (60) an Komponenten durch eine erste Stromversorgung (40) mit Leistung versorgt wird, und mittels Komponenten der ersten Menge (60) ein medizinischer Bildgebungsprozess durchgeführt wird, und
wobei in einem Ruhezustand des Computertomographen (2) eine auf dem Drehkranz (4) angeordnete zweite Menge (62) an Komponenten durch eine von der ersten Stromversorgung (40) schaltungstechnisch getrennte zweite Stromversorgung (50) mit Leistung versorgt wird, und mittels der Komponenten der zweiten Menge (62) das Halbleitermaterial (42) im besagten Gleichgewicht gehalten wird,
**dadurch gekennzeichnet, dass**
die Stromversorgung der zweiten Menge (62) an Komponenten im Ruhezustand über eine von einer ersten Übertragungsstrecke (32) der ersten Stromversorgung (40) schaltungstechnisch trennbare, wenigstens teilweise am Drehkranz (4) angeordnete zweite Übertragungsstrecke (52), welche mit der zweiten Stromversorgung (50) verschaltet ist und einen am Drehkranz (4) angeordneten Anschluss (53) für ein Netzkabel (55) aufweist, erfolgt.

12. Verfahren nach Anspruch 11,
wobei im Betriebszustand eine Temperatur des Halbleitermaterials (42) und/oder eine Bestrahlung des Halbleitermaterials (42) zur Aufrechterhaltung des Gleichgewichts mit einer ersten Genauigkeitskonstante geregelt werden, und
wobei in einem erweiterten Ruhemodus die Temperatur bzw. die Bestrahlung des Halbleitermaterials (42) mit einer zweiten Genauigkeitskonstante geregelt werden, welche größer ist, als die erste Genauigkeitskonstante.

13. Verfahren nach Anspruch 11 oder Anspruch 12,
wobei im Betriebszustand eine Temperatur des Halbleitermaterials (42) und/oder eine Bestrahlung des Halbleitermaterials (42) zur Aufrechterhaltung des Gleichgewichts mit einer ersten Genauigkeitskonstante geregelt werden, und
wobei vor einem Systemneustart des Computertomographen (2) für die Regelung jeweils durch ein entsprechendes, nicht-flüchtig speicherndes Regelelement (68, 69) ein Sollwert und/oder ein Istwert der Temperatur bzw. der Bestrahlung gespeichert wird, und nach dem Systemneustart für die Regelung zur Verfügung gestellt wird.

## Claims

1. Computed tomography device (2) with a holding frame (6) and a ring mount (4), which is mounted movably with respect to the holding frame (4),
wherein the ring mount (4) has an x-ray detector (10) with a semiconductor material (42), which is operable in an equilibrium of statistical states of occupation,
wherein the computed tomography device (2) has a first power supply (40), which is set up to supply power, in an operating state of the computed tomography device (2), to a first lot (60) of components of the computed tomography device (2), which are arranged on the ring mount (4) for an image generation process,
wherein the computed tomography device (2) has a second power supply (50) which can be separated from the first power supply (40) in terms of circuitry and which is set up to supply power to a second lot (62) of components of the computed tomography device (2) in a resting state of the computed tomography device (2), and
wherein the components of the second lot (62) are set up to hold the semiconductor material (42) in the said equilibrium, **characterised by**
a second transmission path (52) which can be separated from a first transmission path (32) of the first power supply (40) in terms of circuitry and is arranged at least partially on the ring mount (4) and is interconnected with the second power supply (50) and has a terminal (53) for a mains cable (55) arranged on the ring mount (4).

2. Computed tomography device (2) according to claim 1, comprising an uninterruptible power supply (56), which can be interconnected with the second lot (62) of components.

3. Computed tomography device (2) according to claim 1 or 2, wherein the second lot (62) of components comprises a first voltage supply (44) for applying a bias voltage to the semiconductor material (42).

4. Computed tomography device (2) according to claim 3, comprising a third power supply (66) which can be separated from the first power supply (40) and the second power supply (50) in terms of circuitry, and which is set up, with a system reboot of the computed tomography device (2), to supply power to the first voltage supply (44) for maintaining the bias voltage on the semiconductor material (42) during the system reboot.

5. Computed tomography device (2) according to claim 3 or claim 4 in conjunction with claim 2,
comprising a voltage buffer (64) connected between the uninterruptible power supply (56) and the first voltage supply (44), which is intended and set up to compensate for a voltage fluctuation with a use of the uninterruptible power supply (56) .

6. Computed tomography device (2) according to one of the preceding claims,
wherein the second lot (62) of components comprises a heating element (45) for heating the semiconductor material (42) and/or a light-emitting element (48) for irradiating the semiconductor material (42).

7. Computed tomography device (2) according to claim 6, wherein the second power supply (50) is configured so that with a system reboot of the computed tomography device (2), the power supply of the second lot (62) of components is interrupted.

8. Computed tomography device according to claim 6 or 7, comprising a first regulation device (47) for regulating a temperature of the semiconductor material (42),
wherein the first regulation device (47) has a first non-volatile-storing regulation element (68), which is set up, with a separation of the first regulation device (47) from an electrical power supply, to store an existing target value and/or an existing actual value of a temperature and with a renewed power supply of the first regulation device (47), to make the stored target value or actual value available in the first regulation device (47).

9. Computed tomography device (2) according to one of claims 6 to 8,
comprising a second regulation device (49) for regulating an irradiation of the semiconductor material (42),
wherein the second regulation device (49) has a second non-volatile-storing regulation element (69), which is set up, with a separation of the second regulation device (49) from an electrical power supply, to store an existing target value and/or an existing actual value of an irradiation and with a renewed electrical power supply of the second regulation device (49), to make the stored target value or actual value available in the second regulation device (49).

10. Computed tomography device (2) according to one of the preceding claims,
wherein the second lot (62) of components in the ring mount (4) is arranged in an electrically isolated manner.

11. Method for operating a computed tomography device (2), which comprises a holding frame (6) and a ring mount (4) mounted movably with respect to the holding frame (6),
wherein the ring mount (4) has an x-ray detector (10) with a semiconductor material (42), which is operable in an equilibrium of statistical states of occupation,
wherein in an operating state of the computed tomography device (2), a first lot (60) of components arranged on the ring mount (4) is supplied with power by means of a first power supply (40) and a medical imaging process is carried out by means of components of the first lot (60), and
wherein in a resting state of the computed tomography device (2), a second lot (62) of components arranged on the ring mount (4) is supplied with power by means of a second power supply (50) which is separated from the first power supply (40) in terms of circuitry, and the semiconductor material (42) is held in the said equilibrium by means of the components of the second lot (62),
**characterised in that**
in the resting state the power supply to the second lot (62) of components takes place by way of a second transmission path (52) which can be separated from a first transmission path (32) of the first power supply (40) in terms of circuitry and is arranged at least partially on the ring mount (4) and is interconnected with the second power supply (50) and has a terminal (53) for a mains cable (55) arranged on the ring mount (4).

12. Method according to claim 11,
wherein in the operating state a temperature of the semiconductor material (42) and/or an irradiation of the semiconductor material (42) are regulated in order to maintain the equilibrium with a first accuracy constant, and
wherein in an extended resting mode, the temperature or the irradiation of the semiconductor material (42) are regulated with a second accuracy constant, which is greater than the first accuracy constant.

13. Method according to claim 11 or claim 12,
wherein in the operating state a temperature of the semiconductor material (42) and/or an irradiation of the semiconductor material (42) are regulated in order to maintain the equilibrium with a first accuracy constant, and
wherein before a system reboot of the computed tomography device (2) for the regulation, a target value and/or an actual value of the temperature or the irradiation is stored by means of a corresponding non-volatile-storing regulation element (68, 69), and made available for the regulation after the system reboot.

## Revendications

1. Tomodensitomètre (2) assisté par ordinateur, comprenant un cadre (6) de maintien et une tourelle (4), qui est montée mobile par rapport au cadre (4) de maintien,
dans lequel la tourelle (4) a un détecteur (10) de rayons X ayant un matériau (42) à semiconducteur, qui est apte à fonctionner dans un équilibre d'états d'occupation statistiques, dans lequel le tomodensitomètre (2) assisté par ordinateur a une première alimentation (40) en courant, qui est agencée pour alimenter en puissance, dans un état de fonctionnement du tomodensitomètre (2) assisté par ordinateur, un premier ensemble (60) de composants du tomodensitomètre (2) assisté par ordinateur, qui sont, pour un processus de production d'images, montés sur la tourelle (4),
dans lequel le tomodensitomètre (2) assisté par ordinateur a une deuxième alimentation (50) en courant, qui peut être séparée en technique de circuit de la première alimentation (40) de courant et qui est agencée pour alimenter en puissance, dans un état de repos du tomodensitomètre (2) assisté par ordinateur, un deuxième ensemble (62) de composants du tomodensitomètre (2) assisté par ordinateur, et
dans lequel les composants du deuxième ensemble (62) sont agencés pour maintenir le matériau (42) semiconducteur dans ledit équilibre,
**caractérisé par**
une deuxième section (52) de transmission, pouvant être séparée en technique de circuit d'une première section (32) de transmission de la première alimentation (40), qui est montée au moins en partie sur la tourelle (4), qui est connectée à la deuxième alimentation (50) en courant et qui a une connexion (53) montée sur la tourelle (4) pour un câble (55) de réseau.

2. Tomodensitomètre (2) assisté par ordinateur suivant la revendication 1,
comprenant une alimentation (56) en courant sans interruption, qui peut être connectée au deuxième ensemble (62) de composants.

3. Tomodensitomètre (2) assisté par ordinateur suivant la revendication 1 ou la revendication 2,
dans lequel le deuxième ensemble (62) de composants comprend une première alimentation (44) en tension pour l'application d'une tension polarisée au matériau (42) semiconducteur.

4. Tomodensitomètre (2) assisté par ordinateur suivant la revendication 3,
comprenant une troisième alimentation (66) en courant, qui peut être séparée en technique de circuit de la première alimentation (40) en courant et de la deuxième alimentation (50) en courant et qui est agencée pour, lors d'un redémarrage du système du tomodensitomètre (2) assisté par ordinateur, alimenter la première alimentation (44) en tension pour le maintien de la tension polarisée appliquée au matériau (42) semiconducteur pendant le redémarrage du système.

5. Tomodensitomètre (2) assisté par ordinateur suivant la revendication 3 ou la revendication 4, en liaison avec la revendication 2,
comprenant un tampon (64) de tension, qui est monté entre l'alimentation (56) en courant sans interruption et la première alimentation (44) en tension et qui est prévu et agencé pour compenser une fluctuation de tension lors de l'utilisation de l'alimentation (56) en courant sans interruption.

6. Tomodensitomètre (2) assisté par ordinateur suivant l'une des revendications précédentes,
dans lequel le deuxième ensemble (62) de composants comprend un élément (45) de chauffage pour chauffer le matériau (42) semiconducteur et/ou un élément (48) d'éclairage pour soumettre le matériau (42) semiconducteur à un rayonnement.

7. Tomodensitomètre (2) assisté par ordinateur suivant la revendication 6,
dans lequel la deuxième alimentation (50) en courant est conformée, de manière à interrompre, lors d'un redémarrage du système du tomodensitomètre (2) assisté par ordinateur, l'alimentation en puissance du deuxième ensemble (62) de composants.

8. Tomodensitomètre assisté par ordinateur suivant la revendication 6 ou la revendication 7,
comprenant un premier dispositif (47) de réglage pour le réglage de la température du matériau (42) semiconducteur,
dans lequel le premier dispositif (47) de réglage a un premier élément (68) de réglage à mémoire non volatile, qui est agencé pour, lors d'une séparation du premier dispositif (47) de réglage d'une alimentation en puissance, mettre en mémoire une valeur de consigne présente et/ou une valeur réelle présente d'une température, et, lors d'une alimentation en puissance renouvelée du premier dispositif (47) de réglage, mettre à disposition du premier dispositif (47) de réglage la valeur de consigne ou la valeur réelle mise en mémoire.

9. Tomodensitomètre (2) assisté par ordinateur suivant l'une des revendications 6 à 8,
comprenant un deuxième dispositif (49) de réglage pour le réglage de l'exposition à un rayonnement du matériau (42) semiconducteur,
dans lequel le deuxième dispositif (49) de réglage a un deuxième élément (69) de réglage à mémoire non volatile, qui est agencé pour, lors d'une séparation du deuxième dispositif (49) de réglage d'une alimentation en puissance, mettre en mémoire une valeur de consigne présente et/ou une valeur réelle présente d'un rayonnement et, lors d'une alimentation en puissance renouvelée du deuxième dispositif (49) de réglage, mettre à disposition du deuxième dispositif (49) de réglage la valeur de consigne ou la valeur réelle mise en mémoire.

10. Tomodensitomètre (2) assisté par ordinateur suivant l'une des revendications précédentes,
dans lequel le deuxième ensemble (62) de composants est monté de manière isolée électriquement dans la tourelle (4).

11. Procédé pour faire fonctionner un tomodensitomètre (2) assisté par ordinateur, qui comprend un premier cadre (6) de maintien et une tourelle (4) montés mobile par rapport au cadre (6) de maintien,
dans lequel la tourelle (4) a un détecteur (10) de rayons X ayant un matériau (42) semiconducteur, qui est apte à fonctionner dans un équilibre d'états d'occupation statistiques, dans lequel dans un état de fonctionnement du tomodensitomètre (2) assisté par ordinateur, on alimente en puissance par une première alimentation (40) en courant un premier ensemble (60), monté sur la tourelle (4), de composants et au moyen de composants du premier ensemble (60), on effectue un processus d'imagerie médicale, et
dans un état de repos du tomodensitomètre (2) assisté par ordinateur, on alimente en puissance par une deuxième alimentation (50) en courant séparée en technique de circuit de la première alimentation (40) en courant, un deuxième ensemble (62), monté sur la tourelle (4), de composants et au moyen des composants du deuxième ensemble (62), on maintient le matériau (42) semiconducteur dans ledit équilibre,
**caractérisé en ce que**
l'alimentation en courant du deuxième ensemble (62) de composants s'effectue dans l'état de repos par une deuxième section (52) de transmission pouvant être séparée en technique de circuit d'une première section (32) de transmission de la première alimentation (40) en courant et montée en partie sur la tourelle (4), qui est connectée à la deuxième alimentation (50) de courant et qui a une connexion (53), montée sur la tourelle (4), pour un câble (55) de réseau.

12. Procédé suivant la revendication 11,
dans lequel, dans l'état de fonctionnement, on règle avec une première constante de précision une température du matériau (42) semiconducteur et/ou une exposition au rayonnement du matériau (42) semiconducteur pour le maintien de l'équilibre, et
dans lequel, dans un mode de repos étendu, on règle la température ou l'exposition au rayonnement du matériau (42) semiconducteur avec une deuxième constante de précision, qui est plus grande que la première constante de précision.

13. Procédé suivant la revendication 11 ou la revendication 12,
dans lequel, dans l'état de fonctionnement, on règle avec une première constante de précision une température du matériau (42) semiconducteur et/ou une exposition au rayonnement du matériau (42) semiconducteur pour maintenir l'équilibre, et
dans lequel avant un redémarrage du système du tomodensitomètre (2) assisté par ordinateur, on met en mémoire pour la régulation respectivement par un élément (68, 69) de réglage adéquat à mémoire non volatile, une valeur de consigne et/ou une valeur réelle de la température ou de l'exposition au rayonnement et on la met à disposition de la régulation après le redémarrage du système.
